# EUROPEAN PATENT SPECIFICATION

(11) **EP 2 583 751 B1**
(45) Date of publication and mention of the grant of the patent: **03.12.2014**
(21) Application number: 11185710.8
(22) Date of filing: 19.10.2011
(51) Int. Cl.: B01J 23/44, B01J 37/02, C07C 5/09, B01J 31/02, C07C 7/167

(54) **Catalyst composition for selective hydrogenation with improved characteristics**
Katalysatorzusammensetzung zur selektiven Hydrierung mit verbesserten Merkmalen
Composition de catalyseur pour l'hydrogénation sélective avec des caractéristiques améliorées

(43) Date of publication of application: 24.04.2013
(73) Proprietor: Clariant Produkte (Deutschland) GmbH, 65929 Frankfurt am Main (DE)
(72) Inventor: Szesni, Normen, Dr., 83022 Rosenheim (DE); Fischer, Richard, Dr., 83043 Bad Aibling (DE); Hagemeyer, Alfred, Dr., Sunnyvale, CA 94086 (US); Großmann, Frank, Dr., 81667 München (DE); Boyer, Jennifer, Prospect, KY 40059 (US); Hou, Hongyi C., San Jose, CA 95126 (US); Lowe, David Michael, Sunnyvale, CA 94086 (US); Lugmair, Claus, San Jose, CA 95136 (US); Sun, Mingyong, Louisville, KY 40241 (US); Urbancic, Michael, Louisville, KY 40241 (US)

(56) References cited:
- EP-A1- 0 064 301
- WO-A2-2011/050953
- TANJA HERRMANN ET AL: "High-performance supported catalysts with an ionic liquid layer for the selective hydrogenation of acetylene", CHEMICAL COMMUNICATIONS, vol. 47, no. 45, 1 January 2011 (2011-01-01), page 12310, XP55013881, ISSN: 1359-7345, DOI: 10.1039/c1cc15325k
- KIWI-MINSKER L ET AL: "Structured catalytic wall microreactor for efficient performance of exothermic reactions", CHEMICAL ENGINEERING AND PROCESSING, ELSEVIER SEQUOIA, LAUSANNE, CH, vol. 49, no. 9, 1 September 2010 (2010-09-01), pages 973-978, XP027382313, ISSN: 0255-2701 [retrieved on 2010-04-28]
- ARRAS ET AL: "Regioselective catalytic hydrogenation of citral with ionic liquids as reaction modifiers", GREEN CHEMISTRY, ROYAL SOCIETY OF CHEMISTRY, CAMBRIDGE, GB, vol. 11, 1 January 2009 (2009-01-01), pages 716-723, XP009150913, ISSN: 1463-9262

## Description

### FIELD OF THE INVENTION

The present invention is related to a catalyst composition for selective hydrogenations, for example for the selective hydrogenation of acetylene in the gaseous phase, comprising a heterogeneous catalyst with a BET surface area of ≤ 9 m²/g and an ionic liquid applied to the surface of the same. The catalyst composition has improved characteristics such as, for example, improved selectivity in favor of the desired product and better thermal stability.

### BACKGROUND OF THE INVENTION

Ethylene and propylene are important monomers for the production of plastics, such as for example polyethylene or polypropylene. Ethylene and propylene are primarily derived from petroleum and petroleum products by means of thermal or catalytic cracking of hydrocarbons. The ethylene or propylene derived with the aid of the cracking process does, however, contain an undesirably high proportion of acetylenic compounds such as acetylene or methyl acetylene (propyne), which can negatively influence downstream ethylene or propylene polymerization. Therefore prior to polymerization the ethylene or propylene must be freed from acetylenic compounds as far as possible.

Typically for the polymerization of ethylene the acetylene concentration must, for example, be reduced to a value of below 1 ppm. For this the acetylene is selectively hydrogenated into ethylene. High requirements are placed on the catalyst and the hydrogenation process. On the one hand, the acetylene must be removed as completely as possible by transformation into ethylene, while the hydrogenation of ethylene into ethane must be prevented, hence the term "selective hydrogenation". In order to ensure this result, the hydrogenation is carried out within a temperature range that is delimited by the so-called "clean-up" temperature and the so-called "run-away" temperature. In the present context the "clean-up" temperature is understood as the temperature from which an appreciable hydrogenation of acetylene into ethylene is observed, while "run-away" temperature is understood as the temperature at which an appreciable hydrogenation of ethylene into ethane commences. The said temperatures can be determined in that the hydrogen consumption of a defined gas mixture containing acetylene, ethylene, and hydrogen is, for example, measured depending on the temperature.

Palladium shell catalysts, often using silver as a promoter, are primarily used as commercial catalysts for the selective hydrogenation of acetylene into ethylene in hydrocarbon streams. The palladium and the silver are supported on an inert, temperature-resistant substrate. The production of these catalysts is carried out in such a way that suitable salts of palladium and silver, for example palladium nitrate and silver nitrate, are applied to a substrate in form of an aqueous solution (impregnation). The impregnation can take place during separate steps with a palladium compound solution and a silver compound solution. It is, however, also possible to apply the solution of palladium compounds and the solution of silver compounds to the substrate simultaneously during a single impregnation step. The impregnated substrate is then calcined to transform the silver into silver oxide, or the palladium into palladium oxide, and is then subjected to a reduction in order to transfer the catalyst into the active form. During the reaction the silver and palladium are assumed to be transferred into the oxidation state "zero".

DE 31 19 850 A1 describes a method for the selective hydrogenation of a diolefin with at least 4 carbon atoms in a hydrocarbon mixture. Hydrogenation takes place with hydrogen on a catalyst containing palladium and silver. The silver/palladium weight ratio of the catalyst is 0.7:1 to 3:1. The production of the catalyst is by way of co-impregnation of a substrate with an aqueous solution of palladium and silver salts.

US 5,648,576 A describes a method for the selective gaseous phase hydrogenation of acetylenic hydrocarbons (C₂-C₃) into the corresponding ethylenic hydrocarbons. The production of the catalyst is realized by co-impregnating the substrate with an aqueous solution of the respective metal salts.

EP 0 064 301 A1 offers a catalyst for the selective gaseous phase hydrogenation of acetylene. The production of the catalyst is realized by means of a two-step application of palladium and silver.

EP 0 780 155 A1 describes the production of hydrogenation catalysts, whereby solutions of palladium nitrate and silver nitrate in a nitrogenous acid are used for the impregnation of the substrate.

Apart from the Pd/Ag catalysts described above, a number of further palladium based catalyst are described, which also provide improved selectivity and sometimes also improved activity; the same include Pd/Zn, Pd/Cd, Pd/Ga and Pd/Au. The latter catalyst family is characterized primarily by a high "run-away" temperature.

According to the definition of Wasserscheid and Keim in "Angewandte Chemie" 2000, 112, pages 3926-3945, ionic liquids are salts, i.e. compounds of anions and cations that are externally neutral, which melt at low temperatures, usually at temperatures of below 100 °C. Ionic liquids are therefore already liquid at low temperatures. In addition they are generally not flammable and have an extremely low vapor pressure. Due to the high variation range of the structure of their cations and anions, their physical and chemical characteristics can be varied over a broad range.

The concept of coating heterogeneous catalysts with small quantities of an ionic liquid has already been described by Jess et al. and Claus et al. [U. Kernchen, B. Etzold, W. Korth, A. Jess, Chem. Eng. Technol. 2007, 30, 985-994; J. Arras, M. Steffan, Y. Shayeghi, P. Claus, Chem. Commun. 2008, 4058-4060]. In both cases an improved selectivity towards the desired product in the target reaction of the hydrogenation of citral or the hydrogenation of diolefins could be achieved than is possible with the uncoated catalyst. This catalyst family has also been named as SCILL - Solid Catalyst with Ionic Liquid Layer - catalysts by the authors.

US 2008/0269533 A1 describes the selective mono-hydrogenation of conjugated dienes with the aid of supported Pd nanoparticles coated with ionic liquids.

International patent application WO2007/124 896 relates to heterogeneous catalysts having a BET surface area of preferably 10 to 300 m²/g. These catalysts may be covered with an ionic liquid and are used for the selective hydrogenation of unsaturated cyclic compounds.

A catalyst system for the selective hydrogenation of acetylene in the simultaneous presence of ethylene comprising a heterogeneous catalyst coated with ionic liquid has also already been described [M. Ruta, G. Laurenczy, P. J. Dyson, L. Kiwi-Minsker, J. Phys. Chem. C 2008, 112, 17814-17819]. However, these catalysts are prepared with support materials that are not suitable for industrial use, as the production of the same is too costly. The described turnovers are also far from realizable.

WO 2011/050953 describes a method for producing a composite material, which comprises a carrier material and an ionic liquid and the use thereof as a synthesis catalyst.

With all of the examples described so far support materials with a high specific surface area and a suitable pore volume were used. In order to achieve an even coating of the entire catalyst surface, and thus the best possible effect (selectivity increase etc.) a relatively large quantity of ionic liquid is required (10 - 17 wt. % in relation to the initial weight of the heterogeneous catalyst). This often results in a substantial pore filling of the catalyst and the reduced activity connected with the same. Ionic liquids are also expensive, which results in substantial additional costs for the overall catalyst formulation.

### SUMMARY OF THE INVENTION

There remains a need for further improving the selectivity of Pd/promoter catalysts for the hydrogenation of acetylenic hydrocarbons, while maintaining or even increasing catalyst activity.

It is therefore the object of this invention to provide a catalyst with high selectivity and activity for the hydrogenation of acetylenic hydrocarbons.

Surprisingly it has been found that conventional heterogeneous catalysts with a BET surface area of ≤ 9 m²/g which are coated with a small amount of an ionic liquid (IL) have improved characteristics, such as improved selectivity in the hydrogenation of unsaturated hydrocarbons while retaining high activity.

With the catalyst system of the invention, known pre-formulated catalysts for the transformation of acetylene into ethylene with a BET surface area of ≤ 9 m²/g are coated with one (or more) ionic liquid(s). The resulting catalyst formulations have a very high selectivity during the hydrogenation of acetylene in ethylene rich gas streams and are further surprisingly characterized by a higher "run-away" temperature. The catalyst formulations of the invention further may use very small quantities of ionic liquid (in the range of ≤ 3% by weight of the catalyst) to achieve these advantageous effects. The loss of catalyst activity is very small.

### DETAILED DESCRIPTION OF THE INVENTION

The pre-formulated catalysts used for coating are, as already described above, supported palladium shell catalysts which preferably comprise at least one further promoter such as for example silver, gold, zinc, tin, lead, gallium, cadmium, copper, bismuth, or potassium. Preferred promoters are silver, gold and zinc, especially silver. Preferred metal or metal-alloy shell thicknesses are between 100 and 500 µm. The Pd metal content in relation to the total weight of the catalyst without ionic liquid is preferably between 10 and 1000 ppm, more preferably between 50 and 500 ppm. For the desired target reaction the catalysts are used either as shaped bodies such as for example tablets, rings, tri-holes, extrudates etc., or as a granulate or powder. The weight ratio of palladium to promoter metal for example lies within a range of 1:5 to 3:1, preferably within a range of 1:4 to 2:1, and particularly preferably within a range of 1:3 to 1:1.

Suitable carrier substrates are Al₂O₃, SiO₂, alumo silicates, TiO₂, ZrO₂, ZnO, MgO, Fe₂O₃ and CeO₂, or mixtures thereof. In order to increase activity or selectivity the substrates can further be doped with at least one of the following elements: Li, Na, K, Rb, Cs, Be, Mg, Ca, Sr and/or Ba. Na, K and/or Ca are particularly suitable.

The BET surface area of the uncoated catalyst is ≤ 9 m²/g, preferably in a range of 1 to 9 m²/g, more preferably in a range of 2 to 8 m²/g, and particularly preferably in a range of 3 to 5 m²/g. The determination of the surface area may be carried out in accordance with ASTM D3663, Standard Test Method for Surface Area of Catalysts and Catalyst Carriers.

The integral pore volume of the catalyst (determined according to DIN 66134 of February 1998 (N₂ adsorption)) without the IL-coating preferably is in the range of 0.005 to 0.07 ml/g, more preferably in the range of 0.007 to 0.04 ml/g and particularly preferably within a range of 0.009 to 0.02 ml/g.

Suitable pre-formulated catalysts for use in preparing supported ionic liquid phase catalyst compositions of the invention include any commercially-available supported Pd or Pd/Ag catalysts supplied by, for example Süd-Chemie, AG, Munich, Germany, BASF, Johnson-Mathey, etc.

For the production of a catalyst composition of the invention a pre-formulated catalyst is loaded with ionic liquid. The ionic liquid to be used for this is not particularly restricted, and in principle, all known ionic liquids suitable for this purpose can be used. Preferred ionic liquids for use with this invention are compounds with the formula (I):

[A]ₙ⁺[Y]ⁿ⁻ (I),

wherein:
n = 1 or 2;
[Y]ⁿ⁻ is selected from the group consisting of tetrafluoroborate ([BF₄]⁻), hexafluorophosphate ([PF₆]⁻), dicyanamide ([N(CN)₂]⁻), halides (Cl⁻, Br⁻, F⁻, I⁻), hexafluoroantimonate ([SbF₆]⁻), nitrate ([NO₃]⁻), nitrite ([NO₂]⁻), anionic metal complexes such as for example [CuCl₄]²⁻, [PdCl₄]²⁻ or [AuCl₄]⁻, acetate ([CH₃COO]⁻), trifluoracetate ([F₃CCOO]⁻), hexafluoroarsenate ([AsF₆]⁻), sulfate ([SO₄]²⁻), hydrogen sulfate ([HSO₄]⁻), alkyl sulfates ([R'-SO₄]⁻), tosylate ([C₇H₇SO₃]⁻), triflate ([CF₃SO₃]⁻), nonaflate ([C₄F₉SO₃]⁻), triperfluoroethylene trifluorophosphate ([PF₃(C₂F₅)₃]⁻), tricyanomethide ([C(CN)₃]⁻), tetracyanoborate ([B(CN)₄]⁻, thiocyanate ([SCN]⁻), carbonate ([CO₃]²⁻), carboxylates ([R'-COO]⁻), sulfonates ([R'SO₃]⁻), dialkylphosphates ([R'PO₄R"]⁻), alkyl phosphonates ([R'HPO₃]⁻) and bissulfonylimides ([(R'-SO₂)₂N]⁻), such as bis(trifluormethylsulfonyl)imide,
wherein R' and R" are the same or different, and each represents a linear or branched, 1 to 12 carbon atom-containing aliphatic or alicyclic alkyl group or a C₅-C₁₈-aryl, C₅-C₁₈-aryl-C₁-C₆-alkyl, or C₁-C₆-alkyl-C₅-C₁₈-aryl group that can be substituted with halogen atoms; and
[A]⁺ is selected from the group consisting of quaternary ammonium cations with the formula [NR¹R²R³R]⁺, phosphonium cations with the formula [PR¹R²R³R]⁺, sulfonium cations with the formula [SR¹R²R]⁺, guadinium cations with the formula (II): 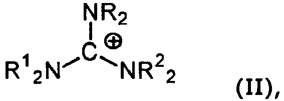 imidazolium cations with the formula (III) 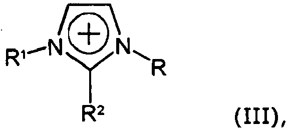 wherein the imidazole core may additionally be substituted with one or more groups selected from C₁-C₆-alkyl, C₁-C₆-alkoxy, C₁-C₆-aminoalkyl, C₅-C₁₂-aryl, and C₅-C₁₂-aryl-C₁-C₆-alkyl groups,
pyridinium cations with the formula (IV) 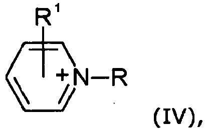 wherein the pyridine core may additionally be substituted with one or more groups selected from C₁-C₆-alkyl, C₁-C₆-alkoxy, C₁-C₆-aminoalkyl, C₅-C₁₂-aryl, and C₅-C₁₂-aryl-C₁-C₆-alkyl groups,
pyrazolium cations with the formula (V) 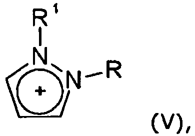 wherein the pyrazole core may additionally be substituted with one or more groups selected from C₁-C₆-alkyl, C₁-C₆-alkoxy, C₁-C₆-aminoalkyl, C₅-C₁₂-aryl, and C₅-C₁₂-aryl-C₁-C₆-alkyl groups,
triazolium cations with the formula (VI) 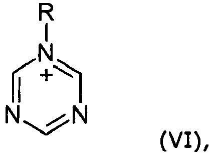 wherein the triazole core may additionally be substituted with one or more groups selected from C₁-C₆-alkyl, C₁-C₆-alkoxy, C₁-C₆-aminoalkyl, C₅-C₁₂-aryl, and C₅-C₁₂-aryl-C₁-C₆-alkyl groups,
and pyrrolidinium cations with the formula (VII) 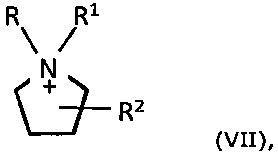 wherein the pyrrolidinium core may additionally be substituted with one or more groups selected from C₁-C₆-alkyl, C₁-C₆-alkoxy, C₁-C₆-aminoalkyl, C₅-C₁₂-aryl, and C₅-C₁₂-aryl-C₁-C₆-alkyl groups,
wherein R¹, R², and R³ are selected independently from each other from the group consisting of: hydrogen; linear or branched, saturated or unsaturated, aliphatic or alicyclic alkyl groups with 1 to 20 carbon atoms, which may be interrupted by one or two of NH, O and/or S; heteroaryl groups with 3 to 8 carbon atoms and at least one hetero atom selected from N, O and S, wherein the heteroaryl groups can be substituted with one or more groups selected from C₁-C₆-alkyl groups and halogen atoms; heteroaryl-C₁-C₆-alkyl groups with 3 to 8 carbon atoms and at least one hetero atom selected from N, O and S in the heteroaryl moiety, wherein the heteroaryl moiety can be substituted with at least one group selected from C₁-C₆-alkyl groups and halogen atoms; polyethers with the formula [-CH₂CH₂O]ₙR^{a} with n = 1 to 50,000, wherein R^{a} is selected from the group consisting of linear or branched, saturated or unsaturated, aliphatic or alicyclic alkyl groups with 1 to 20 carbon atoms; aryl groups with 5 to 12 carbon atoms, which may be substituted with one or more C₁-C₆-alkyl groups and/or halogen atoms; aryl-C₁-C₆-alkyl groups with 5 to 12 carbon atoms in the aryl moiety, which may be substituted with one or more C₁-C₆-alkyl groups and/or halogen atoms, and
wherein R is selected from the group consisting of: linear or branched, saturated or unsaturated, aliphatic or alicyclic alkyl groups with 1 to 20 carbon atoms; heteroaryl-C₁-C₆-alkyl groups with 4 to 8 carbon atoms and at least one hetero atom selected from N, O and S in the heteroaryl moiety, which may be substituted with one or more C₁-C₆-alkyl groups and/or halogen atoms; and aryl-C₁-C₆-alkyl groups with 4 to 12 carbon atoms in the aryl moiety, which may be substituted with one or more C₁-C₆-alkyl groups and/or halogen atoms.

Further preferred ionic liquids for use with this invention are compounds with the formula (I):

[A]ₙ⁺ [Y]ⁿ⁻ (I),

wherein:
n and [Y]ⁿ⁻ are as defined above, and
[A]⁺ is selected from the group consisting of quaternary ammonium cations with the formula [NR¹R²R³R]⁺, imidazolium cations with the formula (III) 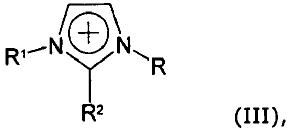 pyridinium cations with the formula (IV) 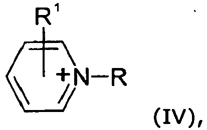 and pyrrolidinium cations with the formula (VII) 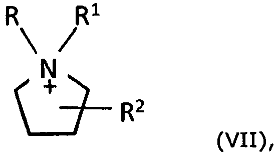
wherein R, R¹, R² and R³ are selected independently from each other from the group consisting of hydrogen; linear or branched C₁-C₁₂-alkyl groups; linear or branched (C₁-C₆-alkyloxy)-C₁-C₆-alkyl groups; and aryl-C₁-C₆-alkyl groups with 5 to 12 carbon atoms in the aryl moiety, which may be substituted with one or more C₁-C₆-alkyl groups and/or halogen atoms.

More preferred ionic liquids for preparing supported ionic liquid phase catalysts of the invention include 1-butyl-3-methylimidazolium triflate, 1-ethyl-3-methylpyridinium ethylsulfate, 1-butyl-1-methylpyrrolidinium triflate, 1-butyl-2,3-dimethylimidazolium triflate, 1-butyl-3-methylimidazolium tricyanomethane, 1-butyl-3-methylimidazolium methylsulfate, 1-butyl-3-methylimidazolium octylsulfate, 1-butyl-3-methylimidazolium tetrafluoroborate, 1-ethyl-3-methylimidazolium ethylsulfate, 1-ethyl-3-methylimidazolium methylphosphonate, 1-ethyl-3-methylimidazolium triflate, 1-butyl-1-methylpyrrolidinium bis(trifluoromethylsufonyl)imide, 1-butyl-1-methylpyrrolidinium tetracyanoborate, 1-butyl-1-methylpyrrolidinium tris(pentafluoroethyl)trifluorophosphate, 1-butyl-3-methylimidazolium bis(trifluoromethylsufonyl)imide, 1-butyl-3-methylimidazolium tricyanomethane, 1-ethyl-3-methylpyridinium bis(trifluoromethylsufonyl)imide, 1-ethyl-3-methylimidazolium tetracyanoborate, 1-ethyl-3-methylimidazolium tris(pentafluoroethyl)trifluorophosphate, 1-ethyl-3-methylpyridinium bis(trifluoromethylsufonyl)imide, 1-methyl-3-octylimidazolium triflate, ethyldimethyl-(2-methoxyethyl)ammonium tris(pentafluoroethyl)trifluorophosphate, tributylmethylammonium dicyanamide, tricyclohexyltetradecylphosphonium tris(pentafluoroethyl)trifluorophosphate, 1-ethyl-3-methylimidazolium bis(trifluoromethylsufonyl)imide, and mixtures thereof.

More preferred ionic liquids further include those of the formula (I), wherein [A]⁺ is selected from the group consisting of 1-butyl-1-methylpyrrolidinium, 1-butyl-2,3-dimethylimidazolium, 1-butyl-3-methylimidazolium, 1-ethyl-3-methylimidazolium, 1-ethyl-3-methylpyridinium, 1-methyl-3-octylimidazolium, ethyldimethyl-(2-methoxyethyl)ammonium, tributylmethylammonium, tricyclohexyltetradecylphosphonium, and mixtures thereof, and wherein [Y]ⁿ⁻ is selected from the group consisting of bis(trifluoromethylsufonyl)imide, dicyanamide, ethylsulfate, methylphosphonate, methylsulfate, octylsulfate, tetracyanoborate, tetrafluoroborate, tricyanomethane, triflate, tris(pentafluoroethyl)trifluorophosphate, and mixtures thereof.

For the production of catalyst compositions of the invention, the ionic liquid or mixtures of several ionic liquids are dissolved or suspended in a solution agent suitable for the purpose, such as for example water, alcohols, acetone etc., or in a solution agent mixture, and applied continuously onto the already pre-formed catalyst inside a reaction chamber with the aid of a nozzle. For this the solution agent is continuously removed from the reaction chamber during the process. In order to achieve an even coating of the substrate, the substrate material is continuously fluidized through a process gas in a process known as fluidized bed coating or through impregnation with a solution or suspension. Further suitable coating processes are dip coating or spray application with a spray pistol or a spray drying pistol.

The amount of ionic liquid relative to the total weight of the catalyst without ionic liquid is for example 0.1 - 10 wt%, preferably 0.2 - 3 wt%, and most preferably 0.3 - 1.5 wt%.

Apart from the application of ionic liquid by means of coating technologies, the same can also be applied by impregnating with a solution or suspension. For this the ionic liquid or mixtures of several ionic liquids are dissolved or suspended in a suitable solution agent (mixture) and subsequently brought into contact with the pre-formed catalyst. The solution agent is then removed under vacuum or at an increased temperature (or both), by resting in air, or by means of a gas stream. The quantity of solution agent used can be equal to or smaller or greater than the pore volume of the catalyst used.

The quantity of ionic liquid used is equal to or smaller than the pore volume of the catalyst used. After the application of the ionic liquid, one is left with an externally dry solid body coated with the desired quantity of ionic liquid. The pore volume of the resulting catalyst composition is reduced by the volume of the ionic liquid. Related to the total weight of the catalyst 0.1 - 5 wt. %, preferably 0.2 - 3 wt. %, and particularly preferably 0.3 - 1.5 wt. % of ionic liquid is used. The distribution of ionic liquid on the macroscopic substrate form body, granulate or powder is freely adjustable by selecting the coating conditions. Depending on the selection of the conditions, a formation of a so-called eggshell, egg-white, egg-yolk, or a uniform distribution of the ionic liquid may result on the substrate. In addition, any concentration gradient of ionic liquid can be created on the substrate. The ionic liquid is preferably applied to the substrate surface as a thin shell. The shell thickness of the ionic liquid on the substrate surface of this invention usually lies within a range of 10 to 2000 µm, preferably within a range of 20 to 1000 µm, and particularly preferably within a range of 50 to 250 µm.

The resulting catalyst can be used without restricting the target reaction. The reduction of metal particles required for activating the catalyst can either take place prior to a coating with the ionic liquid or following the same.

The catalyst can for example be reduced prior to coating with an ionic liquid. The methods to be used for the same are known to the expert, and can for example include wet chemical methods through reduction such as for example NaBH₄, LiAlH₄, hydrazine (hydrate), hypophosphite, formic acid, or salts of the same (formates). In addition a reduction can be brought about in the gaseous phase with hydrogen (in all mixtures with an inert gas; preferably 5% in N₂) within a temperature range of 50-200°C, preferably at 80-120°C.

The reduced metal particles obtained in this way usually have a diameter within a range of 1 to 30 nm, preferably within a range of 1 to 10 nm, and particularly preferably within a range of 2 to 8 nm.

### EXAMPLES

### Example 1

Sample A contains 0.017 wt% Pd on 1-2 mm alumina spheres with a BET surface area of 4.0 m²/g. In order to make Sample A, 1100 g Alpha Alumina was added to 1075 mL PdCl₂ solution (0.178 mg Pd/mL) heated at 70 °C. After the carrier was soaked in the solution for 1 hour, the solution was drained and then the catalyst was washed 10 times using 5 minute soak times with room temperature deionized water. After final wash, the catalyst was calcined in a muffle oven in air at 565 °C for 4 hours.

Sample A1 was made by adding 0.5 wt% of EMIM[EtSO₄] (1-ethyl-3-methylimidazolium ethylsulfate) on Sample A. In order to make Sample A1, Sample A (516.0 mg) was impregnated with an aqueous solution of 1-ethyl-3-methylimidazolium ethylsulfate (232 µL, 11.11 mg/mL) by incipient wetness. The catalyst was dried at 80 °C for 16 hours and reduced at 100 °C in 5% H₂/N₂ for 1 hour.

Sample A2 was made by adding 0.5 wt% of BMIM[OTf] (1-butyl-3-methylimidazolium triflate) on Sample A. In order to make Sample A2, Sample A (476.3 mg) was impregnated with an aqueous solution of 1-butyl-3-methylimidazolium triflate (214 µL, 11.11 mg/mL) by incipient wetness. The catalyst was dried at 80 °C for 16 hours and reduced at 100 °C in 5% H₂/N₂ for 1 hour.

Sample A3 was made by adding 0.5 wt% of BMPr[OTf] (1-butyl-1-methylpyrrolidinium triflate) on Sample A. In order to make Sample A3, Sample A (499.7 mg) was impregnated with an aqueous solution of 1-butyl-1-methylpyrrolidinium triflate (225 µL, 11.11 mg/mL) by incipient wetness. The catalyst was dried at 80 °C for 16 hours and reduced at 100 °C in 5% H₂/N₂ for 1 hour.

Sample A4 was made by adding 0.5 wt% of BMMIM[OTf] (1-Butyl-2,3-dimethylimidazolium triflate) on Sample A. In order to make Sample A4, Sample A (528.8 mg) was impregnated with an aqueous solution of 1-Butyl-2,3-dimethylimidazolium triflate (238 µL, 11.11 mg/mL) by incipient wetness. The catalyst was dried at 80 °C for 16 hours and reduced at 100 °C in 5 % H₂/N₂ for 1 hour.

Sample A5 was made by adding 0.5 wt% of BMIM[BF₄] (1-butyl-3-methylimidazolium tetrafluoroborate) on Sample A. In order to make Sample A5, Sample A (508.2 mg) was impregnated with an aqueous solution of 1-butyl-3-methylimidazolium tetrafluoroborate (229 µL, 11.11 mg/mL) by incipient wetness. The catalyst was dried at 80 °C for 16 hours and reduced at 100 °C in 5% H₂/N₂ for 1 hour.

Sample A6 was made by adding 0.5 wt% of BMIM[MeSO₄] (1-butyl-3-methylimidazolium methylsulfate) on Sample A. In order to make Sample A6, Sample A (511.8 mg) was impregnated with an aqueous solution of 1-butyl-3-methylimidazolium methylsulfate (230 µL, 11.11 mg/mL) by incipient wetness. The catalyst was dried at 80 °C for 16 hours and reduced at 100 °C in 5% H₂/N₂ for 1 hour.

Sample A7 was made by adding 0.5 wt% of BMIM[C₈H₁₇SO₄] (1-butyl-3-methylimidazolium octylsulfate) on Sample A. In order to make Sample A7, Sample A (485.7 mg) was impregnated with an aqueous solution of 1-butyl-3-methylimidazolium octylsulfate (218 µL, 11.11 mg/mL) by incipient wetness. The catalyst was dried at 80 °C for 16 hours and reduced at 100 °C in 5% H₂/N₂ for 1 hour.

Sample A8 was made by adding 0.5 wt% of EMIM[OTf] (1-ethyl-3-methylimidazolium triflate) on Sample A. In order to make Sample A8, Sample A (509.9 mg) was impregnated with an aqueous solution of 1-ethyl-3-methylimidazolium triflate (229 µL, 11.11 mg/mL) by incipient wetness. The catalyst was dried at 80 °C for 16 hours and reduced at 100 °C in 5% H₂/N₂ for 1 hour.

Sample A9 was made by adding 0.5 wt% of EMPy[EtSO₄] (1-ethyl-3-methylpyridinium ethylsulfate) on Sample A. In order to make Sample A9, Sample A (504.0 mg) was impregnated with an aqueous solution of 1-ethyl-3-methylpyridinium ethylsulfate (227 µL, 11.11 mg/mL) by incipient wetness. The catalyst was dried at 80 °C for 16 hours and reduced at 100 °C in 5% H₂/N₂ for 1 hour.

Sample A10 was made by adding 0.5 wt% of EMIM[MePO₃] (1-ethyl-3-methylimidazolium methylphosphonate) on Sample A. In order to make Sample A10, Sample A (517.1 mg) was impregnated with an aqueous solution of 1-ethyl-3-methylimidazolium methylphosphonate (233 µL, 11.11 mg/mL) by incipient wetness. The catalyst was dried at 80 °C for 16 hours and reduced at 100 °C in 5% H₂/N₂ for 1 hour.

Sample A11 was made by adding 0.5 wt% of BMIM[C(CN)₃] (1-butyl-3-methylimidazolium tricyanomethane) on Sample A. In order to make Sample A11, Sample A (504.0 mg) was impregnated with a solution of 1-butyl-3-methylimidazolium bis(trifluoromethylsulfonyl)imide in 2-butanone (227 µL, 11.11 mg/mL) by incipient wetness. The catalyst was dried at 60 °C for 4 hours and reduced at 100 °C in 5% H₂/N₂ for 1 hour.

Sample A12 was made by adding 0.5 wt% of BMIM[NTf₂] (1-butyl-3-methylimidazolium bis(trifluoromethylsulfonyl)imide) on Sample A. In order to make Sample A12, Sample A (513.4 mg) was impregnated with a solution of 1-butyl-3-methylimidazolium bis(trifluoromethylsulfonyl)imide in 2-butanone (231 µL, 11.11 mg/mL) by incipient wetness. The catalyst was dried at 60 °C for 4 hours and reduced at 100 °C in 5% H₂/N₂ for 1 hour.

Sample A13 was made by adding 0.5 wt% of MOIM[OTf] (1-methyl-3-octylimidazolium triflate) on Sample A. In order to make Sample A13, Sample A (502.1 mg) was impregnated with a solution of 1-methyl-3-octylimidazolium triflate in 2-butanone (226 µL, 11.11 mg/mL) by incipient wetness. The catalyst was dried at 60 °C for 4 hours and reduced at 100 °C in 5% H₂/N₂ for 1 hour.

Sample A14 was made by adding 0.5 wt% of EMIM[NTf₂] (1-ethyl-3-methylimidazolium bis(trifluoromethylsulfonyl)imide) on Sample A. In order to make Sample A14, Sample A (490.3 mg) was impregnated with a solution of 1-ethyl-3-methylimidazolium bis(trifluoromethylsulfonyl)imide in 2-butanone (220 µL, 11.11 mg/mL) by incipient wetness. The catalyst was dried at 60 °C for 4 hours and reduced at 100 °C in 5% H₂/N₂ for 1 hour.

Sample A15 was made by adding 0.5 wt% of EMIM[B(CN)₄] (1-ethyl-3-methylimidazolium tetracyanoborate) on Sample A. In order to make Sample A15, Sample A (504.8 mg) was impregnated with a solution of 1-ethyl-3-methylimidazolium tetracyanoborate in 2-butanone (227 µL, 11.11 mg/mL) by incipient wetness. The catalyst was dried at 60 °C for 4 hours and reduced at 100 °C in 5% H₂/N₂ for 1 hour.

Sample A16 was made by adding 0.5 wt% of EMIM[PF₃(C₂F₅)₃] (1-ethyl-3-methylimidazolium tris(pentafluoroethyl)trifluorophosphate) on Sample A. In order to make Sample A16, Sample A (514.4 mg) was impregnated with a solution of 1-ethyl-3-methylimidazolium tris(pentafluoroethyl)trifluorophosphate in 2-butanone (231 µL, 11.11 mg/mL) by incipient wetness. The catalyst was dried at 60 °C for 4 hours and reduced at 100 °C in 5% H₂/N₂ for 1 hour.

Sample A17 was made by adding 0.5 wt% of EMPy[NTf₂] (1-ethyl-3-methylpyridinium bis(trifluoromethylsulfonyl)imide) on Sample A. In order to make Sample A17, Sample A (531.6 mg) was impregnated with a solution of 1-ethyl-3-methylpyridinium bis(trifluoromethylsulfonyl)imide in 2-butanone (239 µL, 11.11 mg/mL) by incipient wetness. The catalyst was dried at 60 °C for 4 hours and reduced at 100 °C in 5% H₂/N₂ for 1 hour.

Sample A18 was made by adding 0.5 wt% of BMPr[NTf₂] (1-butyl-1-methylpyrrolidinium bis(trifluoromethylsulfonyl)imide) on Sample A. In order to make Sample A18, Sample A (512.5 mg) was impregnated with a solution of 1-butyl-1-methylpyrrolidinium bis(trifluoromethylsulfonyl)imide in 2-butanone (230 µL, 11.11 mg/mL) by incipient wetness. The catalyst was dried at 60 °C for 4 hours and reduced at 100 °C in 5% H₂/N₂ for 1 hour.

Sample A19 was made by adding 0.5 wt% of BMPr[PF₃(C₂F₅)₃] (1-butyl-1-methylpyrrolidinium tris(pentafluoroethyl)trifluorophosphate) on Sample A. In order to make Sample A19, Sample A (510.3 mg) was impregnated with a solution of 1-butyl-1-methylpyrrolidinium tris(pentafluoroethyl)trifluorophosphate in 2-butanone (229 µL, 11.11 mg/mL) by incipient wetness. The catalyst was dried at 60 °C for 4 hours and reduced at 100 °C in 5% H₂/N₂ for 1 hour.

Sample A20 was made by adding 0.5 wt% of BMPr[B(CN)₄] (1-butyl-1-methylpyrrolidinium tetracyanoborate) on Sample A. In order to make Sample A20, Sample A (516.0 mg) was impregnated with a solution of 1-butyl-1-methylpyrrolidinium tetracyanoborate in 2-butanone (232 µL, 11.11 mg/mL) by incipient wetness. The catalyst was dried at 60 °C for 4 hours and reduced at 100 °C in 5% H₂/N₂ for 1 hour.

Sample A21 was made by adding 0.5 wt% of TBMA[N(CN)₂] (tributylmethylammonium dicyanamide) on Sample A. In order to make Sample A21, Sample A (474.2 mg) was impregnated with a solution of tributylmethylammonium dicyanamide in 2-butanone (213 µL, 11.11 mg/mL) by incipient wetness. The catalyst was dried at 60 °C for 4 hours and reduced at 100 °C in 5% H₂/N₂ for 1 hour.

Sample A22 was made by adding 0.5 wt% of {EtMe₂(MeOEt)}N[PF₃(C₂F₅)₃] (ethyldimethyl-(2-methoxyethyl)ammonium tris(pentafluoroethyl)trifluorophosphate) on Sample A. In order to make Sample A22, Sample A (477.6 mg) was impregnated with a solution of ethyldimethyl-(2-methoxyethyl)ammonium tris(pentafluoroethyl)trifluorophosphate in 2-butanone (215 µL, 11.11 mg/mL) by incipient wetness. The catalyst was dried at 60 °C for 4 hours and reduced at 100 °C in 5% H₂/N₂ for 1 hour.

### Example 2

Sample B1 was made by adding 0.001 wt% of EMIM[EtSO₄] (1-ethyl-3-methylimidazolium ethylsulfate) on Sample A. In order to make Sample B1, Sample A (485.8 mg) was impregnated with an aqueous solution of 1-ethyl-3-methylimidazolium ethylsulfate (219 µL, 0.022 mg/mL) by incipient wetness. The catalyst was dried at 80°C for 16 hours and reduced at 100 °C in 5% H₂/N₂ for 1 hour.

Sample B2 was made by adding 0.007 wt% of EMIM[EtSO₄] (1-ethyl-3-methylimidazolium ethylsulfate) on Sample A. In order to make Sample B2, Sample A (505.1 mg) was impregnated with an aqueous solution of 1-ethyl-3-methylimidazolium ethylsulfate (227 µL, 0.16 mg/mL) by incipient wetness. The catalyst was dried at 80 °C for 16 hours and reduced at 100 °C in 5 % H₂/N₂ for 1 hour.

Sample B3 was made by adding 0.025 wt% of EMIM[EtSO₄] (1-ethyl-3-methylimidazolium ethylsulfate) on Sample A. In order to make Sample B3, Sample A (512.8 mg) was impregnated with an aqueous solution of 1-ethyl-3-methylimidazolium ethylsulfate (231 µL, 0.56 mg/mL) by incipient wetness. The catalyst was dried at 80 °C for 16 hours and reduced at 100 °C in 5% H₂/N₂ for 1 hour.

Sample B4 was made by adding 0.05 wt% of EMIM[EtSO₄] (1-ethyl-3-methylimidazolium ethylsulfate) on Sample A. In order to make Sample B4, Sample A (468.0 mg) was impregnated with an aqueous solution of 1-ethyl-3-methylimidazolium ethylsulfate (210 µL, 1.11 mg/mL) by incipient wetness. The catalyst was dried at 80 °C for 16 hours and reduced at 100 °C in 5 % H₂/N₂ for 1 hour.

Sample B5 was made by adding 0.1 wt% of EMIM[EtSO₄] (1-ethyl-3-methylimidazolium ethylsulfate) on Sample E. In order to make Sample B5, Sample A (497.3 mg) was impregnated with an aqueous solution of 1-ethyl-3-methylimidazolium ethylsulfate (224 µL, 2.22 mg/mL) by incipient wetness. The catalyst was dried at 80 °C for 16 hours and reduced at 100 °C in 5% H₂/N₂ for 1 hour.

Sample B6 was made by adding 0.25 wt% of EMIM[EtSO4] (1-ethyl-3-methylimidazolium ethylsulfate) on Sample A. In order to make Sample B6, Sample A (480.9 mg) was impregnated with an aqueous solution of 1-ethyl-3-methylimidazolium ethylsulfate (216 µL, 5.56 mg/mL) by incipient wetness. The catalyst was dried at 80 °C for 16 hours and reduced at 100 °C in 5% H₂/N₂ for 1 hour.

### Comparative Example 3

Comparative Sample C contains 0.019 wt% Pd on 1-2 mm alumina spheres with a BET surface area of 50 m²/g. In order to make Comparative Sample C, 10 g alumina was added to 11.4 mL PdCl₂ solution (0.1667 mg Pd/mL) heated at 70 °C. After the carrier was soaked in the solution for 1 hour, the solution was withdrawn and then the catalyst was washed 10 times using 5 minute soak times with room temperature deionized water. After the final washing step, the catalyst was calcined in muffle oven in air at 565 °C for 4 hours.

Comparative Sample C1 was made by adding 0.5 wt% of EMIM[EtSO₄] (1-ethyl-3-methylimidazolium ethylsulfate) on Comparative Sample C. In order to make Comparative Sample C1, Comparative Sample C (502.1 mg) was impregnated with an aqueous solution of 1-ethyl-3-methylimidazolium ethylsulfate (316 µL, 7.94 mg/mL) by incipient wetness. The catalyst was dried at 80 °C for 16 hours and reduced at 100 °C in 5% H₂/N₂ for 1 hour.

Comparative Sample C2 was made by adding 0.5 wt% of BMIM[OTf] (1-butyl-3-methylimidazolium triflate) on Comparative Sample C. In order to make Comparative Sample C2, Comparative Sample C (484.4 mg) was impregnated with an aqueous solution of 1-butyl-3-methylimidazolium triflate (305 µL, 7.94 mg/mL) by incipient wetness. The catalyst was dried at 80 °C for 16 hours and reduced at 100 °C in 5% H₂/N₂ for 1 hour.

### Example 4

Sample A, Samples A1-A22, Samples B1-B6, and Comparative Samples C, C1and C2 were tested as prepared in a microreactor test unit at typical front-end hydrogenation conditions. In the test, a simulated de-propanizer feed containing 0.35 mol% acetylene, 15 mol% hydrogen, 0.02 mol% CO, 47 mol% ethylene, and balance nitrogen was passed over a 260 µl catalyst bed at 478 psig (34 bar) in total pressure and 7000 h⁻¹ in Gas Hourly Space Velocity (GHSV), while the bed temperature was gradually increased from about 45°C. The acetylene concentration at the reactor outlet was monitored with an on-line gas chromatograph (GC). The acetylene concentration at reactor outlet continued decreasing with increasing temperature until reaching <25 ppm. The temperature at this point was defined as the "clean up temperature" (T1). Catalyst bed temperature was further increased until 125°C (the maximum temperature the test unit could reach) or a certain temperature (T2), at which the outlet ethane concentration was >2% due to the increased non-selective reaction of hydrogen with ethylene. The temperature range between T1 and T2 is called the "operation window". Test results of Sample A, Samples A1 to A22, Samples B1-B6, as well as of Comparative Sample C, C1-C2 are listed in the table below. For catalysts that did not run away at the maximum temperature the test unit could reach, T2 was calculated by fitting the data at temperatures above complete acetylene conversion with a first order kinetic model.

### Test Results of Samples A, A1 to A22, B1 to B6, and Comparative Samples C and C1 to C2

| | T1 [°C] | T2 [°C] | Operation Window [°C] | Selectivity at T1 [%] | Ethane Make at 125°C |
|---|---|---|---|---|---|
| Sample A | 63 | 84 | 21 | 92.8 | 10.439 |
| Sample A1 | 68 | 176 | 108 | 96.1 | 0.429 |
| Sample A2 | 68 | 137 | 69 | 96.9 | 0.714 |
| Sample A3 | 61 | 113 | 52 | 89.0 | 3.316 |
| Sample A4 | 62 | 113 | 51 | 85.6 | 3.228 |
| Sample A5 | 69 | 164 | 95 | 94.5 | 0.462 |
| Sample A6 | 68 | 167 | 99 | 97.6 | 0.390 |
| Sample A7 | 68 | 157 | 89 | 95.5 | 0.594 |
| Sample A8 | 65 | 141 | 76 | 96.2 | 1.139 |
| Sample A9 | 65 | 100 | 35 | 95.8 | 9.45 |
| Sample A10 | 82 | 188 | 106 | 90.1 | 0.194 |
| Sample A11 | 68 | 157 | 89 | 90.0 | 0.637 |
| Sample A12 | 61 | 100 | 39 | 89.5 | 4.668 |
| Sample A13 | 67 | 115 | 48 | 66.1 | 3.005 |
| Sample A14 | 60 | 110 | 50 | 99.8 | 3.477 |
| Sample A15 | 71 | 149 | 78 | 90.6 | 0.821 |
| Sample A16 | 67 | 145 | 78 | 90.1 | 1.199 |
| Sample A17 | 62 | 86 | 24 | 93.0 | 10.23 |
| Sample A18 | 64 | 101 | 37 | 88.9 | 4.836 |
| Sample A19 | 62 | 120 | 58 | 74.8 | 2.382 |
| Sample A20 | 69 | 150 | 81 | 98.9 | 0.906 |
| Sample A21 | 65 | 153 | 88 | 85.2 | 0.982 |
| Sample A22 | 66 | 122 | 56 | 93.2 | 2.315 |
| Sample B1 | 62 | 82 | 20 | 47.3 | 10.444 |
| Sample B2 | 63 | 101 | 38 | 69.0 | 8.691 |
| Sample B3 | 60 | 113 | 53 | 96.3 | 9.122 |
| Sample B4 | 61 | 119 | 58 | 95.5 | 5.378 |
| Sample B5 | 65 | 121 | 56 | 98.4 | 3.838 |
| Sample B6 | 67 | 123 | 56 | 100 | 2.428 |
| Comparative Sample C | 56 | 76 | 20 | 91.1 | 10.506 |
| Comparative Sample C1 | 68 | 99 | 31 | 81.3 | 5.725 |
| Comparative Sample C2 | 65 | 98 | 33 | 96.1 | 6.878 |

The operation window as well as the selectivity markedly increase with decrease in BET surface area (Samples A1 and A2 compared to Comparative Samples C1 and C2).

### Example 5

Sample D is a commercial selective hydrogenation catalyst that is supplied by Süd-Chemie AG under trade name OleMax® 251. It contains 0.019 wt% Pd and 0.05 wt% Ag on 4 x 4 mm alumina tablets with a BET surface area of about 4.0 m²/g.

Sample D1 was made by adding 0.5 wt% of BMMIM[OTf] (1-Butyl-2,3-dimethylimidazolium triflate) on Sample D. In order to make Sample D1, 0.6 g of the ionic liquid BMMIM[OTf] were dissolved in 150 ml deionized water. At the same time 120 g of the dry Sample D is fluidized in a reaction chamber with synthetic air as the process gas. The solution of BMMIM[OTf] in water was introduced into the reaction chamber at a flow rate of 5ml/min via a feed pump and sprayed onto the solid catalyst via a spray nozzle at a temperature of 80°C. Once the entire solution has been applied and the substrate is dry, the catalyst formulation is further dried at 80°C for 2 hours.

Sample D2 was made by adding 1.0 wt% of BMMIM[OTf] (1-Butyl-2,3-dimethylimidazolium triflate) on Sample D. In order to make Sample D2, 1.2 g of the ionic liquid BMMIM[OTf] were dissolved in 150 ml deionized water. At the same time 120 g of the dry Sample D is fluidized in a reaction chamber with synthetic air as the process gas. The solution of BMMIM[OTf] in water was introduced into the reaction chamber at a flow rate of 5ml/min via a feed pump and sprayed onto the solid catalyst via a spray nozzle at a temperature of 80°C. Once the entire solution has been applied and the substrate is dry, the catalyst formulation is further dried at 80°C for 2 hours.

Sample D3 was made by adding 2.0 wt% of BMMIM[OTf] (1-Butyl-2,3-dimethylimidazolium triflate) on Sample D. In order to make Sample D3, 2.4 g of the ionic liquid BMMIM[OTf] were dissolved in 150 ml deionized water. At the same time 120 g of the dry Sample D is fluidized in a reaction chamber with synthetic air as the process gas. The solution of BMMIM[OTf] in water was introduced into the reaction chamber at a flow rate of 5ml/min via a feed pump and sprayed onto the solid catalyst via a spray nozzle at a temperature of 80°C. Once the entire solution has been applied and the substrate is dry, the catalyst formulation is further dried at 80°C for 2 hours.

Sample D4 was made by adding 3.0 wt% of BMMIM[OTf] (1-Butyl-2,3-dimethylimidazolium triflate) on Sample D. In order to make Sample D4, 3.6 g of the ionic liquid BMMIM[OTf] were dissolved in 150 ml deionized water. At the same time 120 g of the dry Sample D is fluidized in a reaction chamber with synthetic air as the process gas. The solution of BMMIM[OTf] in water was introduced into the reaction chamber at a flow rate of 5ml/min via a feed pump and sprayed onto the solid catalyst via a spray nozzle at a temperature of 80°C. Once the entire solution has been applied and the substrate is dry, the catalyst formulation is further dried at 80°C for 2 hours.

Sample D1' was made by impregnation of Sample D with a BMMIM[OTf] (1-Butyl-2,3-dimethylimidazolium triflate) solution containing 0.5 g of BMMIM[OTf] in 38 ml deionized water. The clear solution is added to 120 g of dry Sample D. The mixture is then mixed at room temperature for approx. 60 minutes. The catalyst formulation is then dried at 80°C for 16 h to finally obtain Sample D1'.

### Example 6

Samples prepared in Example 5 were tested as prepared in a bench scale test unit at typical front-end hydrogenation conditions. In the test, a simulated de-ethanizer feed containing 0.35 mol% acetylene, 20 mol% hydrogen, 0.02 mol% CO, 45 mol% ethylene, and balance methane was passed over a 25 ml catalyst bed at 500 psig (35.5 bar) in total pressure and 7000 h⁻¹ in Gas Hourly Space Velocity (GHSV), while the bed temperature was gradually increased from about 35°C. The acetylene concentration at the reactor outlet was monitored with an on-line gas chromatograph (GC). The acetylene concentration at reactor outlet continued decreasing with increasing temperature until reaching <25 ppm. The temperature at this point was defined as the "clean up temperature" (T1). Catalyst bed temperature was further increased until 105°C (the maximum temperature the water bath could reach) or a certain temperature (T2), at which the outlet ethane concentration was >2% due to the increased non-selective reaction of hydrogen with ethylene. The temperature range between T1 and T2 is called the "operation window". Test results of Sample D and Samples D1 to D4 and D1' are listed in the table below.

### Front End Deethanizer Feed Test Results

| | T1 [°C] | T2 [°C] | Operation Window [°C] | Selectivity at T1 [%] |
|---|---|---|---|---|
| Sample D | 52 | 57 | 5 | -1 |
| Sample D1 | 61 | 97 | 36 | 52 |
| Sample D2 | 61 | 105 | 44 | 61 |
| Sample D3 | 69 | >105 | >36 | 48 |
| Sample D4 | 73 | >105 | >32 | 56 |
| Sample D1' | 67 | 99 | 32 | 38 |

The operation window as well as the selectivity markedly increase with increasing BMMIM[OTf] content. The optimum BMMIM[OTf] loading seems to be 0.5-1%. At higher loading, the runaway temperature continued to increase at the expense of a higher T1 temperature. Adding BMMIM[OTf] onto Sample D can be realized by coating or wet impregnation; and both methods can generate a new catalyst with significantly improved operation window.

### Example 7

Sample E is a commercial front end selective hydrogenation catalyst that is supplied by Süd-Chemie AG under the trade name OleMax® 250. It contains 0.018 wt% Pd on 4 x 4 mm alumina tablets with a BET surface area of about 4.0 m²/g.

Sample E1 was made by adding 1.0 wt% of BMMIM[OTf] (1-Butyl-2,3-dimethylimidazolium triflate) on Sample E. In order to make Sample E1, 1.2 g of the ionic liquid BMMIM[OTf] were dissolved in 150 ml deionized water. At the same time 120 g of the dry Sample E is fluidized in a reaction chamber with synthetic air as the process gas. The solution of BMMIM[OTf] in water was introduced into the reaction chamber at a flow rate of 5ml/min via a feed pump and sprayed onto the solid catalyst via a spray nozzle at a temperature of 80°C. Once the entire solution has been applied and the substrate is dry, the catalyst formulation is further dried at 80°C for 2 hours.

Sample E2 was made by adding 2.0 wt% of BMMIM[OTf] (1-Butyl-2,3-dimethylimidazolium triflate) on Sample E. In order to make Sample E2, 2.4 g of the ionic liquid BMMIM[OTf] were dissolved in 150 ml deionized water. At the same time 120 g of the dry Sample E is fluidized in a reaction chamber with synthetic air as the process gas. The solution of BMMIM[OTf] in water was introduced into the reaction chamber at a flow rate of 5ml/min via a feed pump and sprayed onto the solid catalyst via a spray nozzle at a temperature of 80°C. Once the entire solution has been applied and the substrate is dry, the catalyst formulation is further dried at 80°C for 2 hours.

Sample E3 was made by adding 3.0 wt% of BMMIM[OTf] (1-Butyl-2,3-dimethylimidazolium triflate) on Sample E. In order to make Sample E3, 3.6 g of the ionic liquid BMMIM[OTf] were dissolved in 150 ml deionized water. At the same time 120 g of the dry Sample E is fluidized in a reaction chamber with synthetic air as the process gas. The solution of BMMIM[OTf] in water was introduced into the reaction chamber at a flow rate of 5ml/min via a feed pump and sprayed onto the solid catalyst via a spray nozzle at a temperature of 80°C. Once the entire solution has been applied and the substrate is dry, the catalyst formulation is further dried at 80°C for 2 hours.

### Example 8

Sample E, Sample E1, Sample E2 and Sample E3 were tested after in-situ reduction at 94°C for 1 hour in a bench scale test unit at typical front-end hydrogenation conditions. In the test, a simulated de-ethanizer feed containing 0.35 mol% acetylene, 20 mol% hydrogen, 0.02 mol% CO, 45 mol% ethylene, and balance methane was passed over a 25 ml catalyst bed at 500 psig (35.5 bar) in total pressure and 7000 h⁻¹ in Gas Hourly Space Velocity (GHSV), while the bed temperature was gradually increased from about 35°C. The acetylene concentration at the reactor outlet was monitored with an on-line gas chromatograph (GC). The acetylene concentration at reactor outlet continued decreasing with increasing temperature until reaching <25 ppm. The temperature at this point was defined as the "clean up temperature" (T1). Catalyst bed temperature was further increased until 105°C (the maximum temperature the water bath could reach) or a certain temperature (T2), at which the outlet ethane concentration was >2% due to the increased non-selective reaction of hydrogen with ethylene. The temperature range between T1 and T2 is called the "operation window". Test results of Sample E and Samples E1 to E3 are listed in the table below.

### Test Results of Sample E and Samples E1 to E3

| | T1 [°C] | T2 [°C] | Operation Window [°C] | Selectivity at T1 [%] |
|---|---|---|---|---|
| Sample E | 53 | 62 | 9 | 58 |
| Sample E1 | 53 | 69 | 16 | 65 |
| Sample E2 | 52 | 81 | 29 | 74 |
| Sample E3 | 62 | 92 | 30 | 78 |

Upon addition of BMMIM[OTf] onto the Pd/alumina catalyst, the operation window increases linearly up to a loading of 2% and then stays constant at 30°C. At higher loading, both T1 and operation window increased.

### Example 9

Comparative Sample F is a commercial selective hydrogenation catalyst that is supplied by Süd-Chemie AG under trade name OleMax® 201. It contains 0.03 wt% Pd and 0.18 wt% Ag on 2-4 mm alumina spheres with a BET surface area of about 35 m²/g.

Comparative Sample F1' was made by adding 0.5 wt% of EMIM[EtSO₄] (1-ethyl-3-methylimidazolium ethylsulfate) onto Sample F by incipient wetness impregnation method. The EMIM[EtSO₄] solution contains 0.5 g of EMIM[EtSO₄] in 60 ml deionized water. The clear solution was added to 100 g of Comparative Sample F and mixed for about 5 min. The catalyst formulation is then dried at 80°C for 16 hr to obtain the final product.

Comparative Sample F2' was made by adding 1.0 wt% of EMIM[EtSO₄] (1-ethyl-3-methylimidazolium ethylsulfate) onto Sample F by incipient wetness impregnation method. The EMIM[EtSO₄] solution contains 1 g of EMIM[EtSO₄] in 60 ml deionized water. The clear solution was added to 100 g of Comparative Sample F and mixed for about 5 min. The catalyst formulation is then dried at 80°C for 16 hr to obtain final product.

Sample D2' was made by adding 0.5 wt% EMIM[EtSO₄] (1-ethyl-3-methylimidazolium ethylsulfate) on Sample D by incipient wetness impregnation. The EMIM[EtSO₄] solution contains 0.5 g of EMIM[EtSO₄] in 24 ml deionized water. The clear solution was added to 100 g of Sample D and mixed for about 5 min. The catalyst formulation is then dried at 80°C for 16 hr to obtain final product.

Sample D3' was made by adding 1 wt% EMIM[EtSO₄] (1-ethyl-3-methylimidazolium ethylsulfate) on Sample D by incipient wetness impregnation. The EMIM[EtSO₄] solution contains 1 g of EMIM[EtSO₄] in 24 ml deionized water. The clear solution was added to 100 g of Sample D and mixed for about 5 min. The catalyst formulation is then dried at 80°C for 16 hr to obtain final product.

### Example 10

Samples and Comparative Samples prepared in Example 9 were tested as prepared in a bench scale test unit at typical front-end hydrogenation conditions. In the test, a simulated de-ethanizer feed containing 0.35 mol% acetylene, 20 mol% hydrogen, 0.02 mol% CO, 45 mol% ethylene, and balance methane was passed over a 25 ml catalyst bed at 500 psig (35.5 bar) in total pressure and 7000 h⁻¹ in Gas Hourly Space Velocity (GHSV), while the bed temperature was gradually increased from about 35°C. The acetylene concentration at the reactor outlet was monitored with an on-line gas chromatograph (GC). The acetylene concentration at reactor outlet continued decreasing with increasing temperature until reaching <25 ppm. The temperature at this point was defined as the "clean up temperature" (T1). Catalyst bed temperature was further increased until 105°C (the maximum temperature the water bath could reach) or a certain temperature (T2), at which the outlet ethane concentration was >2% due to the increased non-selective reaction of hydrogen with ethylene. The temperature range between T1 and T2 is called the "operation window". Test results of Sample F2' and F3' did not run away at the maximum temperature the test unit could reach: the ethane make was 0.35% at 102°C for both catalysts. Their T2's for 2% ethane make were calculated by fitting the data at temperatures above complete acetylene conversion with a first order kinetic model.

### Front End Deethanizer Feed Test Results

| | T1 [°C] | T2 [°C] | T2-T1 [°C] | Selectivity at T1 [%] | Ethane Make at 102°C [%] |
|---|---|---|---|---|---|
| Sample F | 51 | 53 | 2 | -5 | Not operable |
| Sample F1' | 54 | 75 | 21 | 76 | Not operable |
| Sample F2' | 59 | 80 | 21 | 86 | Not operable |
| Sample D | 52 | 57 | 5 | -1 | Not operable |
| Sample D2' | 65 | 148 | 83 | 91 | 0.35 |
| Sample D3' | 66 | 149 | 83 | 94 | 0.35 |

It appears that EMIM[EtSO₄] has much lower impact on Sample F than on Sample D.

## Claims

1. Heterogeneous catalyst, comprising Pd and optionally a promoter, on a support, wherein:
the catalyst is coated with an ionic liquid (IL), and
the BET surface area of the catalyst without the IL-coating is less than or equal to 9 m²/g.

2. The catalyst according to claim 1, wherein the promoter is selected from one or more of silver, gold, zinc, tin, lead, gallium, cadmium, bismuth, potassium and copper.

3. The catalyst according to claim 2, wherein the promoter is silver.

4. The catalyst according to any one of claims 1 to 3, wherein the BET surface area is within a range of 1 to 9 m²/g, preferably within a range of 2 to 8 m²/g, and most preferably within a range of 3 to 5 m²/g.

5. The catalyst according to any one of claims 1 to 4, wherein the integral pore volume of the catalyst without the IL-coating is in the range of 0.005 to 0.07 ml/g, preferably in the range of 0.007 to 0.04 ml/g and more preferably within a range of 0.009 to 0.02 ml/g.

6. The catalyst according to any one of claims 1 to 5, wherein the total weight of Pd relative to the total weight of the catalyst without the IL-coating is between 10 and 1000 ppm, preferably 50 to 500 ppm.

7. The catalyst according to any one of claims 1 to 6, wherein the weight ratio of palladium to promoter is within a range of 1:5 and 3:1, preferably within a range of 1:4 and 2:1, and more preferably within a range of 1:3 and 1:1.

8. The catalyst according to any one of claims 1 to 7, wherein the ionic liquid is a compound of formula (I):
[A]ₙ⁺ [Y]^{n -} (I),
wherein:
n = 1 or 2;
[Y]^{n -} is selected from the group consisting of tetrafluoroborate ([BF₄]⁻), hexafluorophosphate ([PF₆]⁻), dicyanamide ([N(CN)₂]⁻), halides (Cl⁻, Br⁻, F⁻, I⁻), hexafluoroantimonate ([SbF₆]⁻), nitrate ([NO₃]⁻), nitrite ([NO₂]⁻), anionic metal complexes such as [CuCl₄]²⁻, [PdCl₄]²⁻ or [AuCl₄]⁻, acetate ([CH₃COO]⁻), trifluoracetate ([F₃CCOO]⁻), hexafluoroarsenate ([AsF₆]⁻), sulfate ([SO₄]²⁻), hydrogen sulfate ([HSO₄]⁻), alkyl sulfates ([R'-SO₄]⁻), tosylate ([C₇H₇SO₃]⁻), triflate ([CF₃SO₃]⁻), nonaflate ([C₄F₉SO₃]⁻), triperfluoroethylene trifluorophosphate ([PF₃(C₂F₅)₃]⁻), tricyanomethide ([C(CN)₃]⁻), tetracyanoborate ([B(CN)₄]⁻, thiocyanate ([SCN]⁻), carbonate ([CO₃]₂⁻), carboxylates ([R'-COO]⁻), sulfonates ([R'SO₃]⁻), dialkylphosphates ([R'PO₄R"]⁻), alkyl phosphonates ([R'HPO₃]⁻) and bissulfonylimides ([(R'-SO₂)₂N]⁻), such as bis(trifluormethylsulfonyl)imide,
wherein R' and R" are the same or different, and each represents a linear or branched, 1 to 12 carbon atom-containing aliphatic or alicyclic alkyl group or a C₅-C₁₈-aryl, C₅-C₁₈-aryl-C₁-C₆-alkyl, or C₁-C₆-alkyl-C₅-C₁₈-aryl group that can be substituted with halogen atoms; and
[A]⁺ is selected from the group consisting of quaternary ammonium cations with the formula [NR¹R²R³R]⁺, phosphonium cations with the formula [PR¹R²R³R]⁺, sulfonium cations with the formula [SR¹R²R]⁺, guadinium cations with the formula (II): 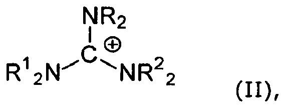 imidazolium cations with the formula (III) 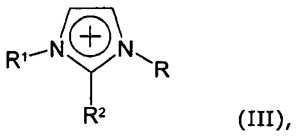 wherein the imidazole core may additionally be substituted with one or more groups selected from C₁-C₆-alkyl, C₁-C₆-alkoxy, C₁-C₆-aminoalkyl, C₅-C₁₂-aryl, and C₅-C₁₂-aryl-C₁-C₆-alkyl groups,
pyridinium cations with the formula (IV) 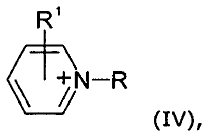 wherein the pyridine core may additionally be substituted with one or more groups selected from C₁-C₆-alkyl, C₁-C₆-alkoxy, C₁-C₆-aminoalkyl, C₅-C₁₂-aryl, and C₅-C₁₂-aryl-C₁-C₆-alkyl groups,
pyrazolium cations with the formula (V) 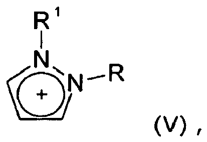 wherein the pyrazole core may additionally be substituted with one or more groups selected from C₁-C₆-alkyl, C₁-C₆-alkoxy, C₁-C₆-aminoalkyl, C₅-C₁₂-aryl, and C₅-C₁₂-aryl-C₁-C₆-alkyl groups,
triazolium cations with the formula (VI) 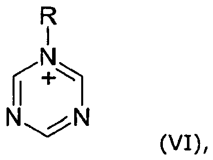 wherein the triazole core may additionally be substituted with one or more groups selected from C₁-C₆-alkyl, C₁-C₆-alkoxy, C₁-C₆-aminoalkyl, C₅-C₁₂-aryl, and C₅-C₁₂-aryl-C₁-C₆-alkyl groups,
and pyrrolidinium cations with the formula (VII) 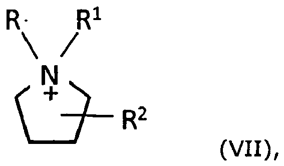 wherein the pyrrolidinium core may additionally be substituted with one or more groups selected from C₁-C₆-alkyl, C₁-C₆-alkoxy, C₁-C₆-aminoalkyl, C₅-C₁₂-aryl, and C₅-C₁₂-aryl-C₁-C₆-alkyl groups,
wherein R¹, R², and R³ are selected independently from each other from the group consisting of: hydrogen; linear or branched, saturated or unsaturated, aliphatic or alicyclic alkyl groups with 1 to 20 carbon atoms, which may be interrupted by one or two of NH, O and/or S; heteroaryl groups with 3 to 8 carbon atoms and at least one hetero atom selected from N, O and S, wherein the heteroaryl groups can be substituted with one or more groups selected from C₁-C₆-alkyl groups and halogen atoms; heteroaryl-C₁-C₆-alkyl groups with 3 to 8 carbon atoms and at least one hetero atom selected from N, O and S in the heteroaryl moiety, wherein the heteroaryl moiety can be substituted with at least one group selected from C₁-C₆-alkyl groups and halogen atoms; polyethers with the formula [-CH₂CH₂O]ₙR^{a} with n = 1 to 50,000, wherein R^{a} is selected from the group consisting of linear or branched, saturated or unsaturated, aliphatic or alicyclic alkyl groups with 1 to 20 carbon atoms; aryl groups with 5 to 12 carbon atoms, which may be substituted with one or more C₁-C₆-alkyl groups and/or halogen atoms; aryl-C₁-C₆-alkyl groups with 5 to 12 carbon atoms in the aryl moiety, which may be substituted with one or more C₁-C₆-alkyl groups and/or halogen atoms, and
wherein R is selected from the group consisting of: linear or branched, saturated or unsaturated, aliphatic or alicyclic alkyl groups with 1 to 20 carbon atoms; heteroaryl-C₁-C₆-alkyl groups with 4 to 8 carbon atoms and at least one hetero atom selected from N, O and S in the heteroaryl moiety, which may be substituted with one or more C₁-C₆-alkyl groups and/or halogen atoms; and aryl-C₁-C₆-alkyl groups with 4 to 12 carbon atoms in the aryl moiety, which may be substituted with one or more C₁-C₆-alkyl groups and/or halogen atoms.

9. The catalyst according to claim 8, wherein the ionic liquid is a compound of formula (I), wherein
[A]⁺ is selected from the group consisting of quaternary ammonium cations with the formula [NR¹R²R³R]⁺, imidazolium cations with the formula (III) 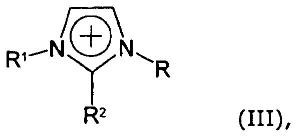 pyridinium cations with the formula (IV) 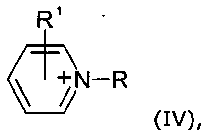 and pyrrolidinium cations with the formula (VII) 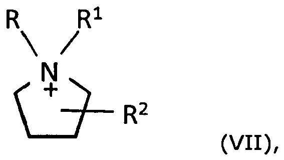 wherein R, R¹, R² and R³ are selected independently from each other from the group consisting of hydrogen; linear or branched C₁-C₁₂-alkyl groups; linear or branched (C₁-C₆-alkyloxy)-C₁-C₆-alkyl groups; and aryl-C₁-C₆-alkyl groups with 5 to 12 carbon atoms in the aryl moiety, which may be substituted with one or more C₁-C₆-alkyl groups and/or halogen atoms.

10. The catalyst according to claim 9, wherein the ionic liquid is selected from 1-butyl-3-methylimidazolium triflate, 1-ethyl-3-methylpyridinium ethylsulfate, 1-butyl-1-methylpyrrolidinium triflate, 1-butyl-2,3-dimethylimidazolium triflate, 1-butyl-3-methylimidazolium tricyanomethane, 1-butyl-3-methylimidazolium methylsulfate, 1-butyl-3-methylimidazolium octylsulfate, 1-butyl-3-methylimidazolium tetrafluoroborate, 1-ethyl-3-methylimidazolium ethylsulfate, 1-ethyl-3-methylimidazolium methylphosphonate, 1-ethyl-3-methylimidazolium triflate, 1-butyl-1-methylpyrrolidinium bis(trifluoromethylsufonyl)imide, 1-butyl-1-methylpyrrolidinium tetracyanoborate, 1-butyl-1-methylpyrrolidinium tris(pentafluoroethyl)trifluorophosphate, 1-butyl-3-methylimidazolium bis(trifluoromethylsufonyl)imide, 1-butyl-3-methylimidazolium tricyanomethane, 1-ethyl-3-methylpyridinium bis(trifluoromethylsufonyl)imide, 1-ethyl-3-methylimidazolium tetracyanoborate, 1-ethyl-3-methylimidazolium tris(pentafluoroethyl)trifluorophosphate, 1-ethyl-3-methylpyridinium bis(trifluoromethylsufonyl)imide, 1-methyl-3-octylimidazolium triflate, ethyldimethyl-(2-methoxyethyl)ammonium tris(pentafluoroethyl)trifluorophosphate, tributylmethylammonium dicyanamide, tricyclohexyltetradecylphosphonium tris(pentafluoroethyl)trifluorophosphate, 1-ethyl-3-methylimidazolium bis(trifluoromethylsufonyl)imide, and mixtures thereof.

11. The catalyst according to any one of claims 1 to 10, wherein the amount of ionic liquid relative to the total weight of the catalyst without ionic liquid is 0.1 - 10 wt%, preferably 0.2 - 3 wt%, and most preferably 0.3 - 1.5 wt%.

12. A method for preparing a catalyst composition according to any one of claims 1 to 11, comprising the steps
a) providing a heterogenous catalyst, comprising Pd and optionally a promoter, on a support, having a BET surface area of less than or equal to 9 m²/g,
b) coating of the catalyst with a mixture of ionic liquid and solvent, and
c) removing the solvent during or after coating the catalyst.

13. The method according to Claim 12, comprising the additional step of reducing the heterogeneous catalyst prior to step b) or after step c).

14. The method according to Claim 12 or 13, wherein the coating step b) is carried out by fluidized bed coating or by a coating through impregnation with a solution or suspension.

15. Use of a catalyst according to any one of the Claims 1 to 11 in the selective hydrogenation of acetylene, preferably in the gas phase or in the liquid phase.

## Patentansprüche

1. Heterogener Katalysator, der Pd und gegebenenfalls einen Promotor umfasst, auf einem Träger, wobei:
der Katalysator mit einer ionischen Flüssigkeit (IF) beschichtet ist und
die BET-Oberfläche des Katalysators ohne die IF-Beschichtung kleiner gleich 9 m²/g ist.

2. Katalysator nach Anspruch 1, wobei der Promotor aus einem oder mehreren der Elemente Silber, Gold, Zink, Zinn, Blei, Gallium, Cadmium, Bismut, Kalium und Kupfer ausgewählt ist.

3. Katalysator nach Anspruch 2, wobei es sich bei dem Promotor um Silber handelt.

4. Katalysator nach einem der Ansprüche 1 bis 3, wobei die BET-Oberfläche im Bereich von 1 bis 9 m²/g, vorzugsweise im Bereich von 2 bis 8 m²/g und ganz besonders bevorzugt im Bereich von 3 bis 5 m²/g liegt.

5. Katalysator nach einem der Ansprüche 1 bis 4, wobei das integrale Porenvolumen des Katalysators ohne die IF-Beschlchtung im Bereich von 0,005 bis 0,07 ml/g, vorzugsweise im Bereich von 0,007 bis 0,04 ml/g und weiter bevorzugt im Bereich von 0,009 bis 0,02 ml/g liegt.

6. Katalysator nach einem der Ansprüche 1 bis 5, wobei das Gesamtgewicht von Pd, bezogen auf das Gesamtgewicht des Katalysators ohne die IF-Beschichtung, zwischen 10 und 1000 ppm liegt und vorzugsweise 50 bis 500 ppm beträgt.

7. Katalysator nach einem der Ansprüche 1 bis 6, wobei das Gewichtsverhältnis von Palladium zu Promotor im Bereich von 1:5 und 3:1, vorzugsweise im Bereich von 1:4 und 2:1 und weiter bevorzugt im Bereich von 1:3 und 1:1 liegt.

8. Katalysator nach einem der Ansprüche 1 bis 7, wobei es sich bei der ionischen Flüssigkeit um eine Verbindung der Formel (I) handelt:
[A]ₙ⁺ [Y]^{n -} (I),
wobei:
n = 1 oder 2;
[Y]^{n -} ausgewählt ist aus der Gruppe bestehend aus Tetrafluoroborat ([BF₄]⁻), Hexafluorophosphat ([PF₆]⁻), Dicyanamid ([N(CN)₂]⁻), Halogeniden (Cl⁻, Br⁻, F⁻, I⁻), Hexafluoroantimonat ([SbF₆]⁻), Nitrat ([NO₃]⁻), Nitrit ([NO₂]⁻), anionischen Metallkomplexen wie [CuCl₄]²⁻ , [PdCl₄]²⁻ oder [AuCl₄]⁻, Acetat ([CH₃COO]⁻), Trifluoracetat ([F₃CCOO]⁻), Hexafluoroarsenat ([AsF₆]⁻), Sulfat ([SO₄]²⁻), Hydrogensulfat ([HSO₄]⁻), Alkylsulfaten ([R'-SO₄]⁻), Tosylat ([C₇H₇SO₃]⁻), Triflat ([CF₃SO₃]⁻), Nonaflat ([C₄F₉SO₃]⁻), Triperfluorethylentrifluorophosphat ([PF₃(C₂F₅)₃]⁻), Tricyanomethid ([C(CN)₃]⁻), Tetracyanoborat ([BCCN)₄]⁻, Thiocyanat ([SCN]⁻), Carbonat ([CO₃]^{2 -}), Carboxylaten ([R'-COO]⁻), Sulfonaten ([R'SO₃]⁻), Dialkylphosphaten ([R'PO₄R'']⁻), Alkylphosphonaten ([R'HPO₃]⁻) und Bissulfonylimiden ([(R'-SO₂)₂N]⁻), wie Bis(trifluormethylsulfonyl)imid,
wobei R' und R'' gleich oder verschieden sind und jeweils für eine lineare oder verzweigte, 1 bis 12 Kohlenstoffatome enthaltende aliphatische oder alicyclische Alkylgruppen oder eine C₅-C₁₈-Aryl-, C₅-C₁₈-Aryl-C₁-C₆-alkyl- oder C₁-C₆-Alkyl-C₅-C₁₈-arylgruppe, die durch Halogenatome substituiert sein kann, stehen; und
[A]⁺ ausgewählt ist aus der Gruppe bestehend aus quaternären Ammoniumkationen mit der Formel [NR¹R²R³R]⁺, Phosphoniumkationen mit der Formel [PR¹R²R³R]⁺, Sulfoniumkationen mit der Formel [SR¹R²R]⁺, Guadiniumkationen mit der Formel (II): 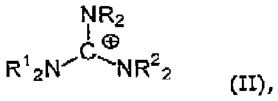
Imidazoliumkationen mit der Formel (III) 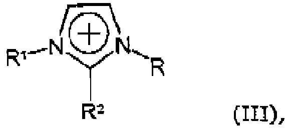 wobei der Imidazolkern zusätzlich durch eine oder mehrere Gruppen, die aus C₁-C₆-Alkyl-, C₁-C₆-Alkoxy-, C₁-C₆-Aminoalkyl-, (C₅-C₁₂-Aryl- und C₅-C₁₂-Aryl-C₁-C₆-alkylgruppen ausgewählt sind, substituiert sein kann,
Pyridiniumkationen mit der Formel (IV) 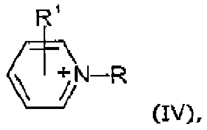 wobei der Pyridinkern zusätzlich durch eine oder mehrere Gruppen, die aus C₁-C₆-Alkyl-, C₁-C₆-Alkoxy-, C₁-C₆-Aminoalkyl-, C₅-C₁₂-Aryl- und C₅-C₁₂-Aryl-C₁-C₆-alkylgruppen ausgewählt sind, substituiert sein kann,
Pyrazoliumkationen mit der Formel (V) 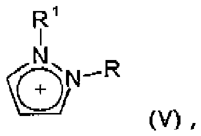 wobei der Pyrazolkern zusätlich durch eine oder mehrere Gruppen, die aus C₁-C₆-Alkyl- C₁-C₆-Alkoxy-, C₁-C₆-Aminoalkyl-, C₅-C₁₂-Aryl- und C₅-C₁₂-Aryl-C₁-C₅-alkylgruppen ausgewählt sind, substituiert sein kann,
Triazoliumkationen mit der Formel (VI) 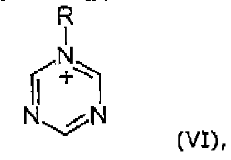 wobei der Triazolkern zusätzlich durch eine oder mehrere Gruppen, die aus C₁-C₆-Alkyl-, C₁-C₆-Alkoxy-, C₁-C₆-Aminoalkyl-, C₅-C₁₂-Aryl- und C₅-C₁₂-Aryl-C₁-C₆-alkylgruppen ausgewählt sind, substituiert sein kann,
und Pyrrolidiniumkationen mit der Formel (VII) 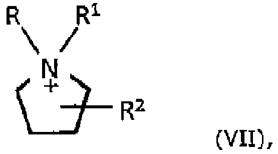 wobei der Pyrrolidiniumkern zusätzlich durch eine oder mehrere Gruppen, die aus C₁-C₆-Alkyl-, C₁-C₆-Alkoxy-, C₁-C₆-Aminoalkyl-, C₅-C₁₂-Aryl- und C₅-C₁₂-Aryl-C₁-C₆-alkylgruppen ausgewählt sind, substituiert sein kann,
wobei R¹, R² und R² unabhängig voneinander ausgewählt sind aus der Gruppe bestehend aus: Wasserstoff; linearen oder verzweigten, gesättigten oder ungesättigten, aliphatischen oder alicyclischen Alkylgruppen mit 1 bis 20 Kohlenstoffatomen, die durch ein oder zwei NH, O und/oder S unterbrochen sein können; Heteroarylgruppen mit 3 bis 8 Kohlenstoffatomen und mindestens einem aus N, O und S ausgewählten Heteroatom, wobei die Heteroarylgruppen durch eine oder mehrere aus C₁-C₆-Alkylgruppen und Halogenatomen ausgewählte Gruppen substituiert sein können; Heteroaryl-C₁-C₆-alkylgruppen mit 3 bis 8 Kohlenstoffatomen und mindestens einem aus N, 0 und 5 ausgewählten Heteroatom in der Heteroarylgruppierung, wobei die Heteroarylgruppierung durch mindestens eine aus C₁-C₆-Alkylgruppen und Halogenatomen ausgewählte Gruppe substituiert sein kann; Polyethern mit der Formel [-CH₂CH₂O]ₙR^{a} mit n = 1 bis 50.000, wobei R^{a} aus der Gruppe bestehend aus linearen oder verzweigten, aliphatischen oder alicyclischen Akylgruppen mit 1 bis 20 Kohlenstoffatomen ausgewahlt ist; Arylgruppe mit 5 bis 12 Kohlenstoffatomen, die durch eine oder mehrere aus C₁-C₆-Alkylgruppen und/oder Halogenatomen ausgewählte Gruppen substituiert sein können; Aryl-C₁-C₆-alkylgruppen mit 5 bis 12 Kohlenstoffatomen in der Arylgruppierung, die durch eine oder mehrere aus C₁-C₆-Alkylgruppen und/oder Halogenatomen ausgewählte Gruppen substituiert sein kann, und
wobei R ausgewählt ist aus der Gruppe bestehend aus: linearen oder verzweigten, gesättigten oder ungesättigten, aliphatischen oder alicyclischen Alkylgruppen mit 1 bis 20 Kohlenstoffatomen; Heteroaryl-C₁-C₆-alkylgruppen mit 4 bis 8 Kohlenstoffatomen und mindestens einem aus N, O und S ausgewählten Heteroatom in der Heteroarylgruppierung, die durch ein oder mehrere C₁-C₆-Alkylgruppen und/oder ein oder mehrere Halogenatome substituiert sein kann; und Aryl-C₁-C₆-alkylgruppen mit 4 bis 12 Kohlenstoffatomen in der Arylgruppierung, die durch eine oder mehrere C₁-C₆-Alkylgruppen und/oder ein oder mehrere Halogenatome substituiert sein kann.

9. Katalysator nach Anspruch 8, wobei es sich bei der ionischen Flüssigkeit um eine Verbindung der Formel (I) handelt, wobei
[A]⁺ ausgewählt ist aus der Gruppe bestehend aus quaternären Ammoniumkationen mit der Formel [NR¹R²R⁵R]⁺, Imidazoliumkationen mit der Formel (III) 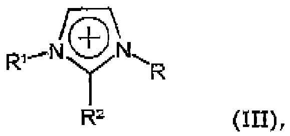
Pyridiniumkationen mit der Formel (IV) 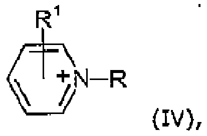
und Pyrrolidiniumkationen mit der Formel (VII) 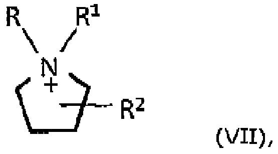
wobei R¹, R² und R³ unabhangig voneinander ausgewählt sind aus der Gruppe bestehend aus: Wasserstoff; linearen oder verzweigten C₁-C₁₂-Alkylgruppen; linearen oder verzweigten (C₁-C₆-Alkyloxy)-C₁-C₆-alkylgruppen und Aryl-C₁-C₆-alkyl-gruppen mit 5 bis 12 Kohlenstoffatomen in der Arylgruppierung, die durch ein oder mehrere C₁-C₆-Alkylgruppen und/oder ein oder mehrere Halogenatome substituiert sein kann.

10. Katalysator nach Anspruch 9, wobei die ionische Flüssigkeit ausgewählt ist aus 1-Butyl-3-methyl-imidazoliumtriflat, 1-Ethyl-3-methylpyridinium-ethylsulfat, 1-Butyl-1-methylpyrrolidiniumtriflat, 1-Butyl-2,3-dimethylimidazoliumtriflat, 1-Butyl-3-methylimidazoliumtricyanomethan, 1-Butyl-3-methyl-imidazoliummethylsulfat, 1-Butyl-3-methyl-imidazoliumoctylsulfat, 1-Butyl-3-methyl-imidazoliumtetrafluoroborat, 1-Ethyl-3-methyl-imidazoliumethylsulfat, 1-Ethyl-3-methyl-imidazoliunmethylphosphonat, 1-Ethyl-3-methyl-imidazoliumtriflat, 1-Butyl-1-methylpyrrolidinium-bis(trifluormethylsufonyl)imid, 1-Butyl-1-methyl-pyrrolidiniumtetracyanoborat, 1-Butyl-1-methyl-pyrrolidiniumtris(pentafluorethyl)trifluorophosphat, 1-Butyl-3-methylimidazoliumbis(trifluormethylsufonyl)imid, 1-Butyl-3-methylimidazolium-tricyanomethan, 1-Ethyl-3-methylpyridiniumbis(trifluormethylsulfonyl)imid, 1-Ethyl-3-methyl-imidazoliumtetracyanoborat, 1-Ethyl-3-methyl-imidazoliumtris(pentafluormethyl)trifluorophosphat, 1-Ethyl-3-methylpyridiniumbis(trifluormethyl-sulfonyl)imid, 1-Methyl-3-octylimidazoliumtriflat, Ethyldimethyl(2-methoxyethyl)ammoniumtris(pentafluorethyl)trifluorophosphat, Tributylmethyl-ammoniumdicyanamid, Tricyclohexyltetradecyl-phosphoniumtris(pentafluorethyl)trifluorophosphat, 1-Ethyl-3-methylimidazoliumbis(trifluormethyl-sufonyl)imid und Mischungen davon.

11. Katalysator nach einem der Ansprüche 1 bis 10, wobei die Menge ionischer Flüssigkeit, bezogen auf das Gesamtgewicht des Katalysators ohne ionische Flüssigkeit, 0,1-10 Gew.-%, vorzugsweise 0,2-3 Gew.-% und ganz besonders bevorzugt 0,3-1,5 Gew.-% beträgt.

12. Verfahren zur Herstellung einer Katalysatorzusammensetzung nach einem der Ansprüche 1 bis 11, das folgende Schritte umfasst:
a) Bereitstellen eines heterogenen Katalysators, der Pd und gegebenenfalls einen Promotor umfasst, auf einem Träger, wobei der Katalysator eine BET-Oberfläche kleiner gleich 9 m²/g aufweist,
b) Beschichten des Katalysators mit einer Mischung von ionischer Flüssigkeit und Lösungsmittel und
c) Entfernen des Lösungsmittels während oder nach dem Beschichten des Katalysators.

13. Verfahren nach Anspruch 12, das den zusätzlichen Schritt der Reduktion des heterogenen Katalysators vor Schritt b) oder nach Schritt c) umfasst.

14. Verfahren nach Anspruch 12 oder 13, bei dem der Beschichtungsschritt b) durch Wirbelschichtbeschichten oder durch Beschichten durch Tränken mit einer Lösung oder Suspension durchgeführt wird.

15. Verwendung eines Katalysators nach einem der Ansprüche 1 bis 11 bei der selektiven Hydrierung von Acetylen, vorzugsweise in der Gasphase oder in der Flüssigphase.

## Revendications

1. Catalyseur hétérogène comprenant Pd et éventuellement un promoteur, sur un support, dans lequel:
le catalyseur est revêtu d'un liquide ionique (IL), et
la surface spécifique BET du catalyseur sans le revêtement d'IL est inférieure ou égale à 9 m²/g.

2. Catalyseur selon la revendication 1, dans lequel le promoteur est choisi parmi un ou plusieurs de l'argent, l'or, le zinc, l'étain, le plomb, le gallium, le cadmium, le bismuth, le potassium et le cuivre.

3. Catalyseur selon la revendication 2, dans lequel le promoteur est l'argent.

4. Catalyseur selon l'une quelconque des revendications 1 à 3, dans lequel la surface spécifique BET est dans une fourchette de 1 à 9 m²/g, de préférence dans une fourchette de 2 à 8 m²/g, et de façon la plus préférée dans une fourchette de 3 à 5 m²/g.

5. Catalyseur selon l'une quelconque des revendications 1 à 4, dans lequel le volume poreux intégral du catalyseur sans le revêtement d'IL est dans la fourchette de 0,005 à 0,07 ml/g, de préférence dans la fourchette de 0,007 à 0,04 ml/g et de façon plus préférée dans une fourchette de 0,009 à 0,02 ml/g.

6. Catalyseur selon l'une quelconque des revendications 1 à 5, dans lequel le poids total de Pd par rapport au poids total du catalyseur sans le revêtement d'IL est compris entre 10 et 1000 ppm, de préférence de 50 à 500 ppm.

7. Catalyseur selon l'une quelconque des revendications 1 à 6, dans lequel le rapport pondéral entre le palladium et le promoteur est dans une fourchette de 1:5 à 3:1, de préférence dans une fourchette de 1:4 à 2:1, et de façon plus préférée dans une fourchette de 1:3 à 1:1.

8. Catalyseur selon l'une quelconque des revendications 1 à 7, dans lequel le liquide ionique est un composé de formule (I) :
[A]ₙ⁺ [Y]^{n -} (I) ,
dans laquelle:
n = 1 ou 2 ;
[Y]^{n -} est choisi dans le groupe constitué par un tétrafluoroborate ([BF₄]⁻), un hexafluorophosphate ([PF₆]⁻), un dicyanamide ([N(CN)₂]⁻), des halogénures (Cl⁻, Br⁻, F⁻, I⁻), un hexafluoroantimonate ([SbF₆]⁻), un nitrate ([NO₃]⁻), un nitrite ([NO₂]⁻), des complexes métalliques anioniques tels que [CuCl₄]²⁻, [PdCl₄]²⁻ ou [AuCl₄]⁻, un acétate ([CH₃COO]⁻), un trifluoracétate ([F₃CCOO]⁻), un hezafluoroarsénate ([AsF₆]⁻), un sulfate ([SO₄]²⁻), un hydrogénosulfate ([HSO₄]⁻), des alkylsulfates ([R'-SO₄]⁻) , un tosylate ([C₇H₇SO₃]⁻), un triflate ([CF₃SO₃]⁻), un nonaflate ([C₄F₉SO₃]⁻), un triperfluoroéthylène-trifluorophosphate ([PF₃(C₂F₅)₃⁻), un tricyanométhylure ([C(CN)₃]⁻), un tétracyanoborate ([B(CN)₄]⁻), un thiocyanate ([SCN]⁻), un carbonate ([CO₃]²⁻), des carboxylates ([R'-COO]⁻), des sulfonates ([R'SO₃]⁻), des dialkylphosphates ([R'PO₄R"]⁻) des alkylphosphonates ([R'HPO₃]⁻) et des bissulfonylimidures ([(R'-SO₂)₂N]⁻), tels que le bis(trifluorométhylsulfonyl)imidure, où R' et R" sont identiques ou différents, et chacun représente un groupe alkyle aliphatique ou alicyclique contenant 1 à 12 atomes de carbone, linéaire ou ramifié, ou un groupe aryle en C₅-C₁₈, aryl (en C₅-C₁₈)-alkyle en C₁-C₆, ou alkyl (en C₁-C₆) -aryle en C₅-C₁₈ qui peut être substitué par des atomes d'halogène ; et
[A]⁺ est choisi dans le groupe constitué par des cations ammonium quaternaire de formule [NR¹R²R³R]⁺, des cations phosphonium de formule [PR¹R²R³R]⁺, des cations sulfonium de formule [SR¹R²R]⁺, des cations guadinium de formule (II) : 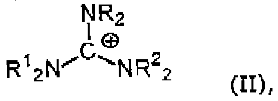
des cations imidazolium de formule (III) 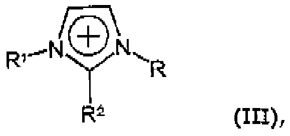 dans laquelle le noyau imidazole peut en plus être substitué par un ou plusieurs groupes choisis parmi les groupes alkyle en C₁-C₆, alcoxy en C₁-C₆, aminoalkyle en C₁-C₆, aryle en C₅-C₁₂ et aryl(en C₅-C₁₂)-alkyle en C₁-C₆,
des cations pyridinium de formule (IV) 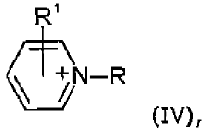 dans laquelle le noyau pyridine peut en plus être substitué par un ou plusieurs groupes choisis parmi les groupes alkyle en C₁-C₆, alcoxy en C₁-C₆, aminoalkyle en C₁-C₆, aryle en C₅-C₁₂ et aryl (en C₅-C₁₂) -alkyle en C₁-C₆,
des cations pyrazolium de formule (V) 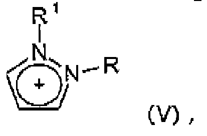 dans laquelle le noyau pyrazole peut en plus être substitué par un ou plusieurs groupes choisis parmi les groupes alkyle en C₁-C₅, alcoxy en C₁-C₆, aminoalkyle en C₁-C₆, aryle en C₅-C₁₂ et aryl (en C₅-C₁₂)-alkyle en C₁-C₆,
des cations triazolium de formule (VI) 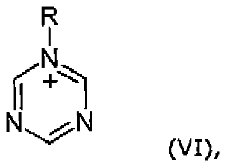 dans laquelle le noyau triazole peut en plus être subssitué par un ou plusieurs groupes choisis parmi les groupes alkyle en C₁-C₆, alcoxy en C₁-C₆, aminoalkyle en C₁-C₆, aryle en C₅-C₁₂ et aryl(en C₅-C₁₂)-alkyle en C₁-C₆,
et des cations pyrrolidinium de formule (VII) 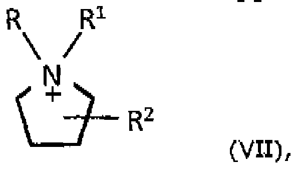 dans laquelle le noyau pyrrolidinium peut en plus être substitué par un ou plusieurs groupes choisis parmi les groupes alkyle en C₁-C₆, alcoxy en C₁-C₆, aminoalkyle en C₁-C₆, aryle en C₅-C₁₂ et aryl(en C₅-C₁₂)-alkyle en C₂-C₆,
dans laquelle R¹, R² et R³ sont choisis indépendamment les uns des autres dans le groupe constitué par : un atome d'hydrogène ; des groupes alkyle aliphatiques ou alicycliques, saturés ou insaturées, linéaires ou ramifiés comportant 1 à 20 atomes de carbone, qui peuvent être interrompus par un ou deux chaînons NH, O et/ou S ; des groupes hétéroaryle comportant 3 à 8 atomes de carbone et au moins un hétéroatome choisi parmi N, O et S, les groupes hétéroaryle pouvant être substitués par un ou plusieurs groupes choisis parmi des groupes alkyle en C₁-C₆ et des atomes d'halogène ; des groupes hétéroaryl-alkyle en C₁-C₆ comportant 3 à 8 atomes de carbone et au moins un hétéroatome choisi parmi N, O et S dans le groupement hétéroaryle, le groupement hétéroaryle pouvant être substitué par au moins un groupe choisi parmi des groupes alkyle en C₁-C₆ et des atomes d'halogène ; des polyéthers de formule [-CH₂CH₂O]ₙR^{a} avec n = 1 à 50 000, où R^{a} est choisi dans le groupe constitué par des groupes alkyle aliphatiques ou alicycliques, saturés ou insaturés, linéaires ou ramifiés comportant 1 à 20 atomes de carbone ; des groupes aryle comportant 5 à 12 atomes de carbone, qui peuvent être substitués par un ou plusieurs groupes alkyle en C₁-C₅ et/ou atomes d'halogène ; des groupes aryl-alkyle en C₁-C₆ comportant 5 à 12 atomes de carbone dans le groupement aryle, qui peuvent être substitués par un ou plusieurs groupes alkyle en C₁-C₆ et/ou atomes d'halogène, et
dans laquelle R est choisi dans le groupe constitué par : des groupes alkyle aliphatiques ou alicycliques, saturés ou insaturés, linéaires ou ramifiés, comportant 1 à 20 atomes de carbone ; des groupes hétèroaryl-alkyle en C₁-C₆ comportant 4 à 8 atomes de carbone et au moins un hétéroatome choisi parmi N, O et S dans le groupement hétéroaryle, qui peuvent être substitués par un ou plusieurs groupes alkyle en C₁-C₆ et/ou atomes d'halogène ; et des groupes aryl-alkyle en C₁-C₆ compotant 4 à 12 atomes de carbone dans le groupement aryle, qui peuvent être substitués par un ou plusieurs groupes alkyle en C₁-C₆ et/ou atomes d'halogène.

9. Catalyseur selon la revendication 8, dans lequel le liquide ionique est un composé de formule (I), dans laquelle
[A]⁺ est choisi dans le groupe constitué par des cations ammonium quaternaire de formule [NR¹R²R³R]⁺, des cations imidazolium de formule (III) 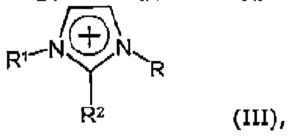
des cations pyridinium de formule (IV) 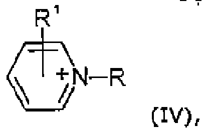
et des cations pyrrolidinium de formule (VII) 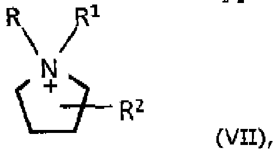 dans laquelle R, R¹, R² et R³ sont choisis indépendamment les uns des autres dans le groupe constitué par un atome d'hydrogène ; des groupes alkyle en C₁-C₁₂ linéaires ou ramifiés ; des groupes alkyloxy(en C₁-C₆)-alkyle en C₁-C₆ linéaires ou ramifiées ; et des groupes aryl-alkyle en C₁-C₆ comportant 5 à 12 atomes de carbone dans le groupement aryle, qui peuvent être substitués par un ou plusieurs groupes alkyle en C₁-C₆ et/ou atomes d'halogéne.

10. Catalyseur selon la revendication 9, dans lequel le liquide ionique est choisi parmi le triflate de 1-butyl-3-méthylimidazolium, l'éthylsulfate de 1-éthyl-3-méthylpyridinium, le triflate de 1-butyl-1-méthylpyrrolidinium, le triflate de 1-butyl-2,3-diméthylimidazolium, le 1-butyl-3-méthylimidazolium-tricyanométhane, le méthylsulfate de 1-butyl-3-méthylimidazolium, l'octylsulfate de 1-butyl-3-méthylimidazolium, le tétrafluoroborate de 1-butyl-3-méthylimidazolium, l'éthylsulfate de 1-éthyl-3-méthylimidazolium, le méthylphosphonate de 1-éthyl-3-méthylimidazolium, le triflate de 1-éthyl-3-méthylimidazolium, le bis (trifluorométhylsulfonyl)imidure de 1-butyl-1-méthylpyrrolidinium, le tétracyanoborate de 1-butyl-1-méthylpyrrolidinium, le tris(pentafluoroéthyl)trifluorophosphate de 1-butyl-1-méthylpyrrolidinium, le bis(trifluorométhylsulfonyl)imidure de 1-butyl-3-méthylimidazolium, le 1-butyl-3-méthylimidazolium-tricyanométhane, le bis-(trifluorométhylsulfonyl)imidure de 1-éthyl-3-méthylpyridinium, le tétracyanoborate de 1-éthyl-3-méthylimidazolium, le tris(pentafluoroéthyl)trifluorophosphate de 1-éthyl-3-méthylimidazolium, le bis(trifluorométhylsulfonyl)imidure de 1-éthyl-3-méthylpyridinium, le triflate de 1-méthyl-3-octylimidazolium, le tris-(pentafluoroéthyl)trifluorophosphate d'éthyldiméthyl-(2-méthoxyéthyl)ammonium, le dicyanamidure de tributylméthylammonium, le tris(pentafluoroéthyl)trifluorophosphate de tricyclohexyltétradécylphosphonium, le bis-(trifluorométhylsulfonyl)imidure de 1-éthyl-3-méthyl-imidazolium, et leurs mélanges.

11. Catalyseur selon l'une quelconque des revendications 1 à 10, dans lequel la quantité de liquide ionique par rapport au poids total du catalyseur sans liquide ionique est de 0,1 - 10 % en poids, de préférence de 0,2 - 3 % en poids, et de façon la plus préférée de 0,3 - 1,5 % en poids.

12. Procédé de préparation d'une composition de catalyseur selon l'une quelconque des revendications 1 à 11, comprenant les étapes consistant à
a) se procurer un catalyseur hétérogène, comprenant Pd et éventuellement un promoteur, sur un support, ayant une surface spécifique BET inférieure ou égale à 9 m²/g,
b) revêtir le catalyseur avec un mélange de liquide ionique et de solvant, et
c) éliminer le solvant pendant ou après le revêtement du catalyseur.

13. Procédé selon la revendication 12, comprenant l'étape supplémentaire consistant à réduire le catalyseur hétérogène avant l'étape b) ou après l'étape c).

14. Procédé selon la revendication 12 ou 13, dans lequel l'étape de revêtement b) est réalisée par revêtement en lit fluidisé ou par un revêtement par imprégnation avec une solution ou une suspension.

15. Utilisation d'un catalyseur selon l'une quelconque des revendications 1 à 11 dans l'hydrogénation sélective de l'acétylène, de préférence en phase gazeuse ou en phase liquide.
